# EUROPEAN PATENT APPLICATION

(11) **EP 1 555 032 A1**
(43) Date of publication of application: **20.07.2005**
(21) Application number: 03765315.1
(22) Date of filing: 17.07.2003
(51) Int. Cl.: A61K 45/06, A61K 31/145, A61K 31/167, A61K 31/17, A61K 31/255, A61K 31/404, A61K 31/405, A61K 31/4365, A61K 31/4439, A61K 31/661, A61K 31/663, A61P 9/00, A61P 9/10

(54) **MEDICINAL COMPOSITION FOR TREATING ARTERIOSCLEROSIS**

(30) Priority: 18.07.2002 JP 2002209165
(71) Applicant: Sankyo Company, Limited, Tokyo 103-8426 (JP)
(72) Inventor: ASAI, Fumitoshi, c/o SANKYO COMPANY, LIMITED, Tokyo 140-8710 (JP); INABA, Toshimori, c/o SANKYO COMPANY, LIMITED, Tokyo 140-8710 (JP); OGAWA, Taketoshi, c/o SANKYO COMPANY, LIMITED, Tokyo 140-8710 (JP)
(74) Representative: Gibson, Christian John Robert
(86) International application number: PCT/JP2003/009108
(87) International publication number: WO 2004/009119

(57) **Abstract**

[Constitution] A medicinal composition **characterized in that** an ADP receptor antagonist and an ACAT inhibitor are administered either simultaneously or separately at a defined interval.

[Advantage] A medicinal composition, wherein an ADP receptor antagonist and an ACAT inhibitor are administered either simultaneously or separately at a defined interval, is useful as a preventive or a remedy (in particular, a remedy) for arteriosclerosis or diseases derived from arteriosclerosis such as ischemic heart disease, ischemic brain disease and peripheral circulation failure in warm-blooded animals (in particular, humans).

## Description

### [Technical Field of the Invention]

The present invention relates to a pharmaceutical composition for prevention or treatment (particularly treatment) of arteriosclerosis or diseases derived from arteriosclerosis (particularly arteriosclerosis) by administering simultaneously, separately or sequentially an adenosine-5'-diphosphate (hereinafter abbreviated as ADP) receptor antagonist and an acyl coenzyme A: cholesterol acyltransferase (hereinafter abbreviated as ACAT) inhibitor.

### [Background of the Invention]

The incidence of atherosclerosis is continuing to increase with the introduction of a Western diet and a growing proportion of elderly people in the population. Since atherosclerosis is the primary cause of myocardial infarction, cerebral infarction, cerebral apoplexy or peripheral vascular disease, there is a need for effective prevention and treatment thereof. Risk factors for atherosclerosis include hyperlipemia (particularly hypercholesterolemia), hypertension and abnormalities in carbohydrate metabolism based on insulin resistance. There are many cases in which these risk factors cause complications (Syndrome X), and their etiology is considered to be mutually interrelated [Diabetes, 37, 1595 (1988)].

Attempts have been made in the past to reduce each of the risk factors of hyperlipemia (particularly hypercholesterolemia), hypertension and abnormalities in carbohydrate metabolism based on insulin resistance for the purpose of preventing or treating atherosclerosis. However, although 3-hydroxy-3-methylglutaryl coenzyme A (hereinafter abbreviated as HMG-CoA) reductase inhibitors ameliorate hyperlipemia, and as a result, demonstrate effectiveness in inhibiting the onset of atherosclerosis, it is known that the effectiveness of a single medicament is not considered to be adequate for patients with serious hyperlipemia or atherosclerosis. Thus, a medicament and a therapeutic method having a new effect are required [Biochim. Biophys. Acta, 960, 294 (1988)].

In the meantime, cholesterol in the diet is known to be absorbed by cells of the intestinal mucosa in the form of free cholesterol where it is esterified by ACAT followed by migration into the blood as cholesterol ester. An ACAT inhibitor is known to inhibit the formation and progression of atherogenic lesions by inhibiting the esterification of cholesterol in foam cells and reducing the accumulation of cholesterol, thereby making it effective against arteriosclerosis.

On the other hand, platelets are also known to be involved in the progression of arteriosclerosis, and platelet aggregation inhibitors are known to be effective against arteriosclerosis. In addition, ADP, which is released from intravascular cells including platelets and damaged blood cells, endothelium or tissue following stimulation by substances like collagen and thrombin, is an example of a platelet activating substance important for platelet activation and aggregation. ADP activates platelets by binding to ADP receptors referred to as P_{2T} receptors. As a result, numerous platelets are bound and stabilized resulting in large platelet aggregates. Platelet ADP receptors that mediate aggregation are members of the P_{2T} receptor family that is activated by purine and/or pyrimidine nucleotides, and are activated by ADP and its derivatives, and are antagonized by adenosine-5'-triphosphate and its derivatives. In addition, research on genetic impairment in humans or rats with reduced release of ADP from platelets or reduced numbers and signaling of ADP receptors has demonstrated that ADP and ADP receptors play an important role in platelet aggregation.

A combination of two or more kinds of well known medicaments having a lipid lowering action is known to be effective in treating hyperlipemia and arteriosclerosis (e.g. The Washington Manual of Medical Therapeutics, 29th Edition, by Department of Medicine, Washington University School of Medicine (1998)). In addition, the efficacy of a combination of lipid lowering agents having new mechanisms of action with an existing medicament has also been pointed out (e.g., Diabete & Metabolisme (Paris), 21, 139 (1995)). However, there are many aspects that remain unclear as to which combination of medicaments makes it possible to obtain an effective and safe preventive or therapeutic agent for hyperlipemia or arteriosclerosis. In addition, since there are also some drugs that are highly toxic (e.g., Drugs of the Future, 25, 171 (2000)), avoiding that toxicity is also considered to be important during concomitant therapy with multiple drugs. Namely, which drugs having certain mechanisms of action can be combined is considered to be the most important factor from both the perspectives of efficacy and safety. The combined use of an ADP receptor antagonist and an ACAT inhibitor being effective for the prevention or treatment of arteriosclerosis has heretofore been unknown.

### [Disclosure of the Invention]

As a result of research in consideration of the importance of prevention and treatment of arteriosclerosis, the inventors of the present invention found that the combined use of an ADP receptor antagonist and an ACAT inhibitor improves inhibitory action on arteriosclerosis in the aorta and inhibitory action on the occurrence of xanthoma in the joints of the extremities, and is useful for the prevention or treatment (particularly treatment) of arteriosclerosis or diseases derived from arteriosclerosis such as ischemic heart disease, ischemic cerebrovascular disease and peripheral vascular disease (particularly arteriosclerosis), thereby leading to completion of the present invention.

An object of the present invention is to provide a pharmaceutical composition for prevention or treatment (particularly treatment) of arteriosclerosis or diseases derived from arteriosclerosis such as ischemic heart disease, ischemic cerebrovascular disease or peripheral vascular disease (particularly arteriosclerosis) for administering simultaneously, separately or sequentially an ADP receptor antagonist and an ACAT inhibitor.

In addition, an object of the present invention is to provide a method for preventing or treating (particularly treating) arteriosclerosis or diseases derived from arteriosclerosis such as ischemic heart disease, ischemic cerebrovascular disease or peripheral vascular disease (particularly arteriosclerosis) by administering to a warm-blooded animal (particularly humans) an effective amount of a pharmaceutical composition for administering simultaneously, separately or sequentially an ADP receptor antagonist and an ACAT inhibitor.

The active ingredients of the pharmaceutical composition of the present invention are an ADP receptor antagonist and an ACAT inhibitor.

Examples of the ADP receptor antagonist that serves as one of the active ingredients of the pharmaceutical composition of the present invention include:
5-[(2-chlorophenyl)methyl]-4,5,6,7-tetrahydrothieno[3,2-c]pyridine described in USP 4,051,141 or USP 4,127,580,
N-[2-(methylthio)ethyl]-2-[(3,3,3-trifluoropropyl)thio]-5'-adenylic acid, monoanhydride with dichloromethylenebisphosphonic acid described in USP 5,955,447 and Journal of Medicinal Chemistry, 1999, Vol. 42, p.213-220,
2-(propylthio)-5'-adenylic acid, monoanhydride with dichloromethylenebis(phosphonic acid) described in Journal of Medicinal Chemistry, 1999, Vol. 42, p.213-220,
methyl (+)-(S)-α-(2-chlorophenyl)-6,7-dihydrothieno[3,2-c]pyridine-5(4H)-acetate described in USP 4,529,596, USP 4,847,265 or USP 5,576,328, or
2-acetoxy-5-(α-cyclopropylcarbonyl-2-fluorobenzyl)-4,5,6,7-tetrahydrothieno[3,2-c]pyridine, or pharmaceutically acceptable salts thereof described in USP 5,288,726 or WO 02/04461,
preferably, 5-[(2-chlorophenyl)methyl]-4,5,6,7-tetrahydrothieno[3,2-c]pyridine (particularly, its hydrochloride), N-[2-(methylthio)ethyl]-2-[(3,3,3-trifluoropropyl)thio]-5'-adenylic acid, monoanhydride with dichloromethylenebisphosphonic acid, methyl (+)-(S)-α-(2-chlorophenyl)-6,7-dihydrothieno[3,2-c]pyridine-5(4H)-acetate (particularly, its sulfate), or 2-acetoxy-5-(α-cyclopropylcarbonyl-2-fluorobenzyl)-4,5,6,7-tetrahydrothieno[3,2-c]pyridine (particularly, its hydrochloride), or pharmaceutically acceptable salts thereof,
more preferably, 2-acetoxy-5-(α-cyclopropylcarbonyl-2-fluorobenzyl)-4,5,6,7-tetrahydrothieno[3,2-c]pyridine, or pharmaceutically acceptable salts (particularly, its hydrochloride) thereof.

Further, the ACAT inhibitor i.e., one of active ingredients of the pharmaceutical composition of this invention is, for example,
(±)-N-(1,2-diphenylethyl)-2-(2-octyloxyphenyl)acetamide described in WO 92/09561,
2,6-bis(1-methylethyl)phenyl N-[[2,4,6-tris(1-methylethyl)phenyl]acetyl]sulfamate described in USP 5,491,172, USP 5,633,287, USP 6,093,719, USP 6,124,309 or USP 6,143,755,
(1S,2S)-2-[N-(2,2-dimethylpropyl)-N-nonylcarbamoyl]aminocyclohexan-1-yl 3-[N-(2,2,5,5-tetramethyl-1,3-dioxane-4-carbonyl)amino]propionate described in USP 5,120,738,
(S)-2',3',5'-trimethyl-4'-hydroxy-α-dodecylthio-α-phenylacetanilide described in USP 5,990,173,
2-[3-(2-cyclohexylethyl)-3-(4-dimethylaminophenyl)ureido]-4-methoxy-6-tert-butylphenol and its hydrochloride described in UPS 5,849,732,
(-)-4-{(4R,5R)-2-[3-(2,6-diisopropylphenyl)ureidomethyl]-4,5-dimethyl-1,3-dioxolan-2-yl}phenylphosphate and its monosodium salt described in WO 96/26948,
N-[2,4-bis(methylthio)-6-methyl-3-pyridyl]-2-[4-[2-(oxazolo[4,5-b]pyridin-2-ylthio)ethyl]piperazin-1-yl]acetamide described in EP 987254,
N-(2,6-diisopropylphenyl)-2-tetradecylthioacetamide described in USP 5,475,130,
trans-1,4-bis[[1-cyclohexyl-3-(4-dimethylaminophenyl)ureido]methyl]cyclohexane described in USP 5,733,931,
1-benzyl-1-[3-(pyrazol-3-yl)benzyl]-3-[2,4-bis(methylthio)-6-methylpyridin-3-yl]urea described in WO 96/10559,
N-(4,6-dimethyl-1-pentylindolin-7-yl)-2,2-dimethylpropanamide described in USP 5,990,150 or USP 6,127,403, or
N-(1-octyl-5-carboxymethyl-4,6-dimethylindolin-7-yl)-2,2-dimethylpropanamide and its sulfate, or pharmaceutically acceptable salts thereof described in USP 6,063,806 or USP 6,200,988,
preferably, 2,6-bis(1-methylethyl)phenyl N-[[2,4,6-tris(1-methylethyl)phenyl]acetyl]sulfamate, (S)-2',3',5'-trimethyl-4'-hydroxy-α-dodecylthio-a-phenylacetanilide, (-)-4-{(4R,5R)-2-[3-(2,6-diisopropylphenyl)ureidomethyl]-4,5-dimethyl-1,3-dioxolan-2-yl}phenylphosphate, N-(2,6-diisopropylphenyl)-2-tetradecylthioacetamide, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(4,6-dimethyl-1-pentylindolin-7-yl)-2,2-dimethylpropanamide, N-(1-octyl-5-carboxymethyl-4,6-dimethylindolin-7-yl)-2,2-dimethylpropanamide, or pharmaceutically acceptable salts thereof,

Further, preferably, (S)-2',3',5'-trimethyl-4'-hydroxy-α-dodecylthio-α-phenylacetanilide, (-)-4-{(4R,5R)-2-[3-(2,6-diisopropylphenyl)ureidomethyl]-4,5-dimethyl-1,3-dioxolan-2-yl}phenylphosphate, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(1-octyl-5-carboxymethyl-4,6-dimethylindolin-7-yl)-2,2-dimethylpropanamide, or pharmaceutically acceptable salts thereof,
more preferably, N-(1-octyl-5-carboxymethyl-4,6-dimethylindolin-7-yl)-2,2-dimethylpropanamide, or pharmaceutically acceptable salts thereof,
most preferably, sulfate of N-(1-octyl-5-carboxymethyl-4,6-dimethylindolin-7-yl)-2,2-dimethylpropanamide (particularly, hemisulfate).

The ADP receptor antagonist and the ACAT inhibitor, which are active ingredients of the pharmaceutical composition of the present invention, can be made to be in the form of pharmaceutically acceptable salts as desired, and these are also included in the present invention.

In the case where the ADP receptor antagonist or the ACAT inhibitor, which are active ingredients of the pharmaceutical composition of the present invention, have a basic group, they can be made to be in the form of acid addition salts in accordance with conventional methods as desired. For example, they can be obtained by treating with the corresponding acid for 5 to 30 minutes at room temperature in a solvent (such as an ether, ester or alcohol, and preferably an ether) followed filtering out the precipitated crystals or distilling off the solvent under reduced pressure. Examples of such salts include inorganic acid salts such as hydrofluorides, hydrochlorides, hydrobromides, hydroiodides, nitrates, perchlorates, sulfates or phosphates; sulfonates such as methanesulfonates, trifluoromethanesulfonates, ethanesulfonates, benzenesulfonates or p-toluenesulfonates; carboxylates such as fumarates, succinates, citrates, tartrates, oxalates or maleates; and amino acid salts such as glutamates or aspartates. In the case where the corresponding acid has a valence of 2 in the aforementioned acid addition salts, any salt in which the molar ratio of the ADP receptor antagonist or the ACAT inhibitor to acid is 1:1 or 2:1 (and preferably 2:1) is included in the present invention, and in the case where the corresponding acid has a valence of 3, any salt in which the aforementioned ratio is 1:1, 2:1 or 3:1 is also included in the present invention.

In addition, in the case where the ADP receptor antagonist and the ACAT inhibitor, which are active ingredients of the pharmaceutical composition of the present invention, has an acidic group, they can be made to be in the form of the respective pharmaceutically acceptable salts by treating with a base in accordance with conventional methods as desired. For example, they can be obtained by treating with the corresponding base for 5 to 30 minutes at room temperature in a solvent (such as an ether, ester or alcohol, and preferably an alcohol) followed by filtering out the precipitated crystals or distilling off the solvent under reduced pressure. Such salts can include, for example, alkali metal salts such as a sodium salt, a potassium salt or a lithium salt, alkaline earth metal salts such as a calcium salt or a magnesium salt, metal salts such as an aluminium salt, an iron salt, a zinc salt, a copper salt, a nickel salt or a cobalt salt; inorganic salts such as an ammonium salt; and organic salts such as a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzylphenethylamine salt, a piperazine salt, a tetramethylammonium salt or a tris(hydroxymethyl)aminomethane salt, preferably, alkali metal salts (particularly, a sodium salt or a potassium salt).

Where stereoisomers may be present, in the case where various geometrical isomers or asymmetric carbons are contained in the ADP receptor antagonist or the ACAT inhibitor, which is an active ingredient of the pharmaceutical composition of the present invention, each of these or mixtures thereof are also included in the present invention.

Where the ADP receptor antagonist or the ACAT inhibitor, which is an active ingredient of the pharmaceutical composition of the present invention, can exist in the form of a hydrate that contains various proportions of water, each of these or mixtures thereof are also included in the present invention.

In the case where the ADP receptor antagonist and the ACAT inhibitor, which are active ingredients of the pharmaceutical composition of the present invention, are administered "simultaneously", there are no particular limitations on the administration form provided they can be administered at nearly the same time. They are preferably administered as a single composition.

In the case where the ADP receptor antagonist and the ACAT inhibitor, which are active ingredients of the pharmaceutical composition of the present invention, are administered "separately or sequentially", there are no particular limitations on the administration form provided they can be administered separately or sequentially. For example, the ADP receptor antagonist may be administered first followed by administration of the ACAT inhibitor after a predetermined amount of time, or the ACAT inhibitor may be administered first followed by administration of the ADP receptor antagonist after a predetermined amount of time.

The pharmaceutical composition of the present invention is preferably:
(1) a pharmaceutical composition in which the ADP receptor antagonist is 5-[(2-chlorophenyl)methyl]-4,5,6,7-tetrahydrothieno[3,2-c]pyridine, N-[2-(methylthio)ethyl]-2-[(3,3,3-trifluoropropyl)thio]-5'-adenylic acid, monoanhydride with dichloromethylenebisphosphonic acid, 2-(propylthio)-5'-adenylic acid, monoanhydride with dichloromethylenebis (phosphonic acid), methyl (+)-(S)-α-(2-chlorophenyl)-6,7-dihydrothieno[3,2-c]pyridine-5(4H)-acetate, 2-acetoxy-5-(α-cyclopropylcarbonyl-2-fluorobenzyl)-4,5,6,7-tetrahydrothieno[3,2-c]pyridine, or pharmaceutically acceptable salts thereof,
(2) a pharmaceutical composition in which the ADP receptor antagonist is 5-[(2-chlorophenyl)methyl]-4,5,6,7-tetrahydrothieno[3,2-c]pyridine·hydrochloride, N-[2-(methylthio)ethyl]-2-[(3,3,3-trifluoropropyl)thio]-5'-adenylic acid, monoanhydride with dichloromethylenebisphosphonic acid, methyl (+)-(S)-α-(2-chlorophenyl)-6,7-dihydrothieno[3,2-c]pyridine-5(4H)-acetate.sulfate, 2-acetoxy-5-(a-cyclopropylcarbonyl-2-fluorobenzyl)-4,5,6,7-tetrahydrothieno[3,2-c]pyridine or 2-acetoxy-5-(α-cyclopropylcarbonyl-2-fluorobenzyl)-4,5,6,7-tetrahydrothieno[3,2-c]pyridine·hydrochloride,
(3) a pharmaceutical composition in which the ADP receptor antagonist is 5-[(2-chlorophenyl)methyl]-4,5,6,7-tetrahydrothieno[3,2-c]pyridine or pharmaceutically acceptable salts thereof,
(4) a pharmaceutical composition in which the ADP receptor antagonist is 5-[(2-chlorophenyl)methyl]-4,5,6,7-tetrahydrothieno[3,2-c]pyridine·hydrochloride,
(5) a pharmaceutical composition in which the ADP receptor antagonist is N-[2-(methylthio)ethyl]-2-[(3,3,3-trifluoropropyl)thio]-5'-adenylic acid, monoanhydride with dichloromethylenebisphosphonic acid or pharmaceutically acceptable salts thereof,
(6) a pharmaceutical composition in which the ADP receptor antagonist is methyl (+)-(S)-α-(2-chlorophenyl)-6,7-dihydrothieno[3,2-c]pyridine-5(4H)-acetate or pharmaceutically acceptable salts thereof,
(7) a pharmaceutical composition in which the ADP receptor antagonist is methyl (+)-(S)-α-(2-chlorophenyl)-6,7-dihydrothieno[3,2-c]pyridine-5(4H)-acetate·sulfate,
(8) a pharmaceutical composition in which the ADP receptor antagonist is 2-acetoxy-5-(α-cyclopropylcarbonyl-2-fluorobenzyl)-4,5,6,7-tetrahydrothieno[3,2-c]pyridine or pharmaceutically acceptable salts thereof,
(9) a pharmaceutical composition in which the ADP receptor antagonist is 2-acetoxy-5-(α-cyclopropylcarbonyl-2-fluorobenzyl)-4,5,6,7-tetrahydrothieno[3,2-c]pyridine or 2-acetoxy-5-(α-cyclopropylcarbonyl-2-fluorobenzyl)-4,5,6,7-tetrahydrothieno[3,2-c]pyridine·hydrochloride,
(10) a pharmaceutical composition in which the ACAT inhibitor is, 2,6-bis(1-methylethyl)phenyl N-[[2,4,6-tris(1-methylethyl)phenyl]acetyl]sulfamate, (S)-2',3',5'-trimethyl-4'-hydroxy-α-dodecylthio-α-phenylacetanilide, (-)-4-{(4R,5R)-2-[3-(2,6-diisopropylphenyl)ureidomethyl]-4,5-dimethyl-1,3-dioxolan-2-yl}phenyl phosphate, N-(2,6-diisopropylphenyl)-2-tetradecylthioacetamide, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(4,6-dimethyl-1-pentylindolin-7-yl)-2,2-dimethylpropanamide, N-(1-octyl-5-carboxymethyl-4,6-dimethylindolin-7-yl)-2,2-dimethylpropanamide, or pharmaceutically acceptable salts thereof,
(11) pharmaceutical composition in which the ACAT inhibitor is (±)-N-(1,2-diphenylethyl)-2-(2-octyloxyphenyl)acetamide, 2,6-bis(1-methylethyl)phenyl N-[[2,4,6-tris(1-methylethyl)phenyl]acetyl]sulfamate, (1S,2S)-2-[N-(2,2-dimethylpropyl)-N-nonylcarbamoyl]aminocyclohexan-1-yl 3-[N-(2,2,5,5-tetramethyl-1,3-dioxane-4-carbonyl)amino]propionate, (S)-2',3',5'-trimethyl-4'-hydroxy-α-dodecylthio-α-phenylacetanilide, 2-[3-(2-cyclohexylethyl)-3-(4-dimethylaminophenyl)ureido]-4-methoxy-6-tert-butylphenol·hydrochloride, (-)-4-{(4R,5R)-2-[3-(2,6-diisopropylphenyl)ureidomethyl]-4,5-dimethyl-1,3-dioxolan-2-yl}phenylphosphate·monosodium salt, N-[2,4-bis(methylthio)-6-methyl-3-pyridyl]-2-[4-[2-(oxazolo[4,5-b]pyridin-2-ylthio)ethyl]piperazin-1-yl]acetamide, N-(2,6-diisopropylphenyl)-2-tetradecylthioacetamide, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(4,6-dimethyl-1-pentylindolin-7-yl)-2,2-dimethylpropanamide or sulfate of N-(1-octyl-5-carboxymethyl-4,6-dimethylindolin-7-yl)-2,2-dimethylpropanamide,
(12) a pharmaceutical composition in which the ACAT inhibitor is (S)-2',3',5'-trimethyl-4'-hydroxy-α-dodecylthio-α-phenylacetanilide, (-)-4-{(4R,5R)-2-[3-(2,6-diisopropyphenyl)ureidomethyl]-4,5-dimethyl-1,3-dioxolan-2-yl}phenyl phosphate, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(1-octyl-5-carboxymethyl-4,6-dimethylindolin-7-yl)-2,2-dimethylpropanamide, or pharmaceutically acceptable salts thereof,
(13) a pharmaceutical composition in which the ACAT inhibitor is N-(1-octyl-5-carboxymethyl-4,6-dimethylindolin-7-yl)-2,2-dimethylpropanamide or pharmaceutically acceptable salts thereof,
(14) a pharmaceutical composition in which the ACAT inhibitor is sulfate of N-(1-octyl-5-carboxymethyl-4,6-dimethylindolin-7-yl)-2,2-dimethylpropanamide, or
(15) a pharmaceutical composition in which the ACAT inhibitor is N-(1-octyl-5-carboxymethyl-4,6-dimethylindolin-7-yl)-2,2-dimethylpropanamide·hemisulfate.
   Further, pharmaceutical compositions obtained by arbitrarily combining an ADP receptor antagonist selected from the above (1) to (9) and an ACAT inhibitor selected from the above (10) to (15) are also preferable and include, for example,
(16) a pharmaceutical composition in which the ADP receptor antagonist is 5-[(2-chlorophenyl)methyl]-4,5,6,7-tetrahydrothieno[3,2-c]pyridine, N-[2-(methylthio)ethyl]-2-[(3,3,3-trifluoropropyl)thio]-5'-adenylic acid, monoanhydride with dichloromethylenebisphosphonic acid, 2-(propylthio)-5'-adenylic acid, monoanhydride with dichloromethylenebis(phosphonic acid), methyl (+)-(S)-α-(2-chlorophenyl)-6,7-dihydrothieno[3,2-c]pyridine-5(4H)-acetate, 2-acetoxy-5-(α-cyclopropylcarbonyl-2-fluorobenzyl)-4,5,6,7-tetrahydrothieno[3,2-c]pyridine, or pharmaceutically acceptable salts thereof, and
   the ACAT inhibitor is 2,6-bis(1-methylethyl)phenyl-N-[[2,4,6-tris(1-methylethyl)phenyl]acetyl]sulfamate, (S)-2',3',5'-trimethyl-4'-hydroxy-α-dodecylthio-α-phenylacetanilide, (-)-4-{(4R,5R)-2-[3-(2,6-diisopropylphenyl)ureidomethyl]-4,5-dimethyl-1,3-dioxolan-2-yl}phenyl phosphate, N-(2,6-diisopropylphenyl)-2-tetradecylthioacetamide, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(4,6-dimethyl-1-pentylindolin-7-yl)-2,2-dimethylpropanamide, N-(1-octyl-5-carboxymethyl-4,6-dimethylindolin-7-yl)-2,2-dimethylpropanamide, or pharmaceutically acceptable salts thereof,
(17) a pharmaceutical composition in which the ADP receptor antagonist is 5-[(2-chlorophenyl)methyl]-4,5,6,7-tetrahydrothieno[3,2-c]pyridine·hydrochloride, N-[2-(methylthio)ethyl]-2-[(3,3,3-trifluoropropyl)thio]-5'-adenylic acid, monoanhydride with dichloromethylenebisphosphonic acid, methyl (+)-(S)-α-(2-chlorophenyl)-6,7-dihydrothieno[3,2-c]pyridine-5(4H)-acetate·sulfate, 2-acetoxy-5-(α-cyclopropylcarbonyl-2-fluorobenzyl)-4,5,6,7-tetrahydrothieno[3,2-c]pyridine or 2-acetoxy-5-(α-cyclopropylcarbonyl-2-fluorobenzyl)-4,5,6,7-tetrahydrothieno[3,2-c]pyridine·hydrochloride, and
   the ACAT inhibitor is (±)-N-(1,2-diphenylethyl)-2-(2-octyloxyphenyl)acetamide, 2,6-bis(1-methylethyl)phenyl N-[[2,4,6-tris(1-methylethyl)phenyl]acetyl] sulfamate, (1S,2S)-2-[N-(2,2-dimethylpropyl)-N-nonylcarbamoyl]aminocyclohexan-1-yl 3-[N-(2,2,5,5-tetramethyl-1,3-dioxane-4-carbonyl)amino]propionate, (S)-2',3',5'-trimethyl-4'-hydroxy-α-dodecylthio-α-phenylacetanilide, 2-[3-(2-cyclohexylethyl)-3-(4-dimethylaminophenyl)ureido]-4-methoxy-6-tertbutylphenol·hydrochloride, (-)-4-{(4R,5R)-2-[3-(2,6-diisopropylphenyl)ureidomethyl]-4,5-dimethyl-1,3-dioxolan-2-yl}phenylphosphate·monosodium salt, N-[2,4-bis(methylthio)-6-methyl-3-pyridyl]-2-[4-[2-(oxazolo[4,5-b]pyridin-2-ylthio)ethyl]piperazin-1-yl]acetamide, N-(2,6-diisopropylphenyl)-2-tetradecylthioacetamide, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(4,6-dimethyl-1-pentylindolin-7-yl)-2,2-dimethylpropanamide or a sulfate of N-(1-octyl-5-carboxymethyl-4,6-dimethylindolin-7-yl)-2,2-dimethylpropanamide,
(18) a pharmaceutical composition in which the ADP receptor antagonist is 5-[(2-chlorophenyl)methyl]-4,5,6,7-tetrahydrothieno[3,2-c]pyridine, or pharmaceutically acceptable salts thereof, and
   the ACAT inhibitor is (S)-2',3',5'-trimethyl-4'-hydroxy-α-dodecylthio-α-phenylacetanilide, (-)-4-{(4R,5R)-2-[3-(2,6-diisopropylphenyl)ureidomethyl]-4,5-dimethyl-1,3-dioxolan-2-yl}phenyl phosphate, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(1-octyl-5-carboxymethyl-4,6-dimethylindolin-7-yl)-2,2-dimethylpropanamide, or pharmaceutically acceptable salts thereof,
(19) a pharmaceutical composition in which the ADP receptor antagonist is methyl (+)-(S)-α-(2-chlorophenyl)-6,7-dihydrothieno[3,2-c]pyridine-5(4H)-acetate or pharmaceutically acceptable salts thereof, and
   the ACAT inhibitor is N-(1-octyl-5-carboxymethyl-4,6-dimethylindolin-7-yl)-2,2-dimethylpropanamide or pharmaceutically acceptable salts thereof,
(20) a pharmaceutical composition in which the ADP receptor antagonist is methyl (+)-(S)-α-(2-chlorophenyl)-6,7-dihydrothieno[3,2-c]pyridine-5(4H)-acetate·sulfate, and
   the ACAT inhibitor is N-(1-octyl-5-carboxymethyl-4,6-dimethylindolin-7-yl)-2,2-dimethylpropanamide or pharmaceutically acceptable salts thereof,
(21) a pharmaceutical composition in which the ADP receptor antagonist is 2-acetoxy-5-(α-cyclopropylcarbonyl-2-fluorobenzyl)-4,5,6,7-tetrahydrothieno[3,2-c]pyridine, or pharmaceutically acceptable salts thereof, and
   the ACAT inhibitor is N-(1-octyl-5-carboxymethyl-4,6-dimethylindolin-7-yl)-2,2-dimthylpropanamide or pharmaceutically acceptable salts thereof,
(22) a pharmaceutical composition in which the ADP receptor antagonist is 2-acetoxy-5-(α-cyclopropylcarbonyl-2-fluorobenzyl)-4,5,6,7-tetrahydrothieno[3,2-c]pyridine or 2-acetoxy-5-(α-cyclopropylcarbonyl-2-fluorobenzyl)-4,5,6,7-tetrahydrothieno[3,2-c]pyridine·hydrochloride, and
   the ACAT inhibitor is a sulfate of N-(1-octyl-5-carboxymethyl-4,6-dimethylindolin-7-yl)-2,2-dimethylpropanamide, and more preferably,
(23) a pharmaceutical composition in which the ADP receptor antagonist is 2-acetoxy-5-(α-cyclopropylcarbonyl-2-fluorobenzyl)-4,5,6,7-tetrahydrothieno[3,2-c]pyridine or 2-acetoxy-5-(α-cyclopropylcarbonyl-2-fluorobenzyl)-4,5,6,7-tetrahydrothieno[3,2-c]pyridine·hydrochloride, and
   the ACAT inhibitor is N-(1-octyl-5-carboxymethyl-4,6-dimethylindolin-7-yl)-2,2-dimethylpropanamide·hemisulfate.
   In addition, the present invention is preferably:
(24) a method for preventing or treating arteriosclerosis or diseases derived from arteriosclerosis by administering to a warm-blooded animal an effective amount of a pharmaceutical composition according to the aforementioned (1) to (23), or
(25) a method according to the aforementioned (24)
   wherein the warm-blooded animal is a human.

### [Mode for carrying out the Invention]

The ADP receptor antagonist, an active ingredient of the pharmaceutical composition of the present invention, can be easily prepared according to the methods described in USP 4,051,141, USP 4,127,580, USP 5,955,447, Journal of Medicinal Chemistry, 1999, Vol. 42, p.213-220, USP 5,721,219, USP 4,529,596, USP 4,847,265, USP 5,576,328, USP 5,288,726 or WO 02/04461 or the analogous methods thereto.

Further, the ACAT inhibitor, an active ingredient of the pharmaceutical composition of the present invention can be easily prepared according to methods described in WO 92/09561, USP 5,491,172, USP 5,633,287, USP 6,093,719, USP 6,124,309, USP 6,143,755, USP 5,120,738, USP 5,990,173, USP 5,849,732, WO 96/26948, EP 987254, USP 5,475,130, USP 5,733,931, WO 96/10559, USP 5,990,150, USP 6,127,403, USP 6,063,806 or USP 6,200,988 or the analogous methods thereto.

The pharmaceutical composition of the present invention for simultaneous, separate or sequential administration of an ADP receptor antagonist and an ACAT inhibitor has superior inhibitory action on the occurrence of arteriosclerosis in the aorta and on the occurrence of xanthoma in the joints of the extremities, and has low toxicity. It is useful as a preventive or therapeutic agent (particularly a therapeutic agent) for arteriosclerosis or diseases derived from arteriosclerosis such as ischemic heart disease, ischemic cerebrovascular disease or peripheral vascular disease (particularly arteriosclerosis) in warm-blooded animals (particularly humans). It should be noted that the combined use of the ADP receptor antagonist and the ACAT inhibitor that are active ingredients of the present invention demonstrates superior effects as compared with the use of either ingredient alone.

The ADP receptor antagonist and the ACAT inhibitor that are the active ingredients of the pharmaceutical composition of the present invention can be prepared separately in individual unit dosage forms or they can be mixed and physically prepared into a single unit dosage form.

In the case of using the pharmaceutical composition of the present invention as a preventive or therapeutic agent for the aforementioned diseases, the ADP receptor antagonist and the ACAT inhibitor that are the active ingredients of the composition of the present invention can themselves be administered, or they can be mixed with a suitable, pharmaceutically acceptable vehicle, diluent and so forth, and then administered orally in the form of a preparation such as tablets, capsules, granules, powder or syrup, or administered parenterally in the form of a preparation such as an injection or suppository.

These preparations can be produced by well known methods using additives such as a excipients (for example, organic excipients including sugar derivatives such as lactose, sucrose, glucose, mannitol or sorbitol; starch derivatives such as corn starch, potato starch, α-starch or dextrin; cellulose derivatives such as crystalline cellulose; gum arabic; dextran; and Pullulan; and inorganic excipients including silicate derivatives such as light silicic acid anhydride, synthesized aluminum silicate, calcium silicate and magnesium aluminate metasilicate; phosphates such as calcium hydrogen phosphate; carbonates such as calcium carbonate; and sulfates such as calcium sulfate), lubricants (for example, stearic acid and stearic acid metal salts such as calcium stearate and magnesium stearate; talc; colloidal silica; waxes such as beeswax and spermaceti wax; boric acid; adipic acid; sulfates such as sodium sulfate; glycol; fumaric acid; sodium benzoate; DL leucine; laurylsulfates such as sodium laurylsulfate and magnesium laurylsulfate; silicic acid derivatives such as silicic acid anhydride and silicic acid hydrate; and the aforementioned starch derivatives), binders (for example, hydroxypropyl cellulose, hydroxypropylmethyl cellulose, polyvinylpyrrolidone, macrogol, the above excipients and similar compounds, disintegrants (for example, cellulose derivatives such as low-substituted hydroxypropyl cellulose, carboxymethyl cellulose, calcium carboxymethyl cellulose and internally bridged sodium carboxymethyl cellulose; and chemically-modified starches and celluloses such as carboxymethyl starch, sodium carboxymethyl starch and internally bridged polyvinylpyrrolidone), emulsifiers (for example, colloidal clay such as bentonite and veegum; metal hydroxides such as magnesium hydroxide and aluminium hydroxide; anionic surfactants such as sodium laurylsulfate and calcium stearate; cationic surfactants such as benzalkonium chloride; and nonionic surfactants such as polyoxyethylene alkyl ether, polyoxyethylene sorbitan fatty acid ester and sucrose fatty acid ester), stabilizers (for example, parahydroxybenzoate esters such as methyl p-hydroxybenzoate and propyl p-hydroxybenzoate; alcohols such as chlorobutanol, benzyl alcohol and phenylethyl alcohol; benzalkonium chloride; phenols such as phenol and cresol; thimerosal; dehydroacetic acid; and sorbic acid), flavour and odour correctives (for example, normally used sweeteners, sour flavourings and fragrances) and diluents.

The dose and dose ratio of the ADP receptor antagonist and the ACAT inhibitor that are the active ingredients of the pharmaceutical composition of the present invention can be varied according to various conditions such as the individual drug activity as well as patient condition, age and body weight.

Although varying according to the patient's condition, age and so forth, the adult dose in the case of oral administration has a lower limit per dosage of 0.1 mg (preferably 0.5 mg) and an upper limit per dosage of 1000 mg (preferably 500 mg), and in the case of parenteral administration, a lower limit per dosage of 0.01 mg (preferably 0.05 mg) and an upper limit per dosage of 100 mg (preferably 50 mg), and is administered from one to six times per day according to symptoms either simultaneously, separately or sequentially.

It should be noted that the dose of the ADP receptor antagonist in the case of using for prevention or treatment of arteriosclerosis in the present invention can be lower than the dose in the case of using as a blood coagulation inhibitor, which is the inherent application, and the dose can be further lowered due to the superior effects resulting from combined use with the ACAT inhibitor.

In addition, the ratio of the doses of the ADP receptor antagonist to the ACAT inhibitor that are the active ingredients of the pharmaceutical composition of the present invention can be, for example, within a range of 1:10000 to 10000:1, preferably 1:1000 to 1000:1, and more preferably 1:100 to 100:1, on the basis of weight, although it can be varied over a wide range,

Particularly, a ratio of dose of 2-acetoxy-5-(α-cyclopropylcarbonyl-2-fluorobenzyl)-4,5,6,7-tetrahydrothieno[3,2-c]pyridine or 2-acetoxy-5-(α-cyclopropylcarbonyl-2-fluorobenzyl)-4,5,6,7-tetrahydrothieno[3,2-c]pyridine·hydrochloride, to a sulfate (preferably, hemisulfate) of N-(1-octyl-5-carboxymethyl-4,6-dimethylindolin-7-yl)-2,2-dimethylpropanamide is preferably 1:200 - 200:1 on the basis of weight.

### [Best Mode for carrying out the Invention]

Whilst the following provides a more detailed explanation of the present invention through its Examples and Preparation examples, the scope of the present invention is not limited thereto.

### Example 1

A test was conducted in which male New Zealand White rabbits (body weights: 1.0 to 1.5 kg) were loaded for 10 weeks with a cholesterol diet (0.5% cholesterol, 3% peanut oil and 3% coconut oil) in the amounts indicated below. The animals were fed 40 g for the first two weeks, 50 g for the next four weeks, and 60 g for the final four weeks. Two weeks after cholesterol diet loading, a Nylon thread was aseptically inserted from the right femoral artery to the vicinity of the diaphragm to induce chronic endothelial damage in the abdominal aorta and right femoral artery. The Nylon thread was immobilized for 8 weeks at the insertion site. Following surgery to insert the Nylon thread, the animals were grouped so that the average values of serum total cholesterol, triglycerides, platelet aggregation and body weight of each group were nearly equal, followed by oral administration of the ADP receptor antagonist and the ACAT inhibitor either alone or combined. After necrotomy of the animals 8 weeks after administration, the right femoral arteries were excised and the ratio of the surface area covered by atherogenic lesions was calculated. After exposing the right femoral artery (by 1 cm from the branch of the iliac artery), it was photographed with a digital camera (COOLPX990, Nikon) followed by measurement of the surface area of the lumen and the surface area covered by atherogenic lesions using an image analyzer, and calculation of the ratio of the surface area covered by atherogenic lesions relative to the surface area of the aorta lumen as a percentage. In addition, a section 1 cm from the branch of the iliac artery on the side of the femoral artery was cut out and after fixing in methanol-carnoy solution, was embedded in a paraffin block. After slicing into thin sections, the sections were immunostained using Elastica-Masson stain and anti-human α-actin antibody (1A4) followed by calculating the degree of medial intimal thickness and the smooth muscle cell occupancy rate using an image analyzer.

The results are shown in Table 1. In the table, Compound A represent 2-acetoxy-5-(α-cyclopropylcarbonyl-2-fluorobenzyl)-4,5,6,7-tetrahydrothieno[3,2-c]pyridine, and Compound B represents sulfate of N-(1-octyl-5-carboxymethyl-4,6-dimethylindolin-7-yl)-2,2-dimethylpropanamide.

**[Table 1]**

| Group administered | Ratio of lesion surface area (%) | Medial intimal thickness (%) | Smooth muscle cell occupancy rate (%) |
|---|---|---|---|
| Control group | 63.6 ± 15.4 | 266.6 ± 120.5 | 8.5 ± 5.3 |
| Compound A (1 mg/kg) | 47.2 ± 14.2 | 275.4 ± 153.4 | 5.5 ± 1.5 |
| Compound B (3 mg/kg) | 40.6 ± 19.8* | 60.0 ± 34.2** | 25.6 ± 14.8* |
| Compound A (1 mg/kg) + Compound B (3 mg/kg) | 24.2 ± 16.6** | 46.3 ± 30.1** | 31.2 ± 15.9* |

| | | | |
|---|---|---|---|
| *: Significant difference is less than 5% with respect to control group as determined with Dunnett's multiple comparison test | | | |
| **: Significant difference is less than 1% with respect to control group as determined with Dunnett's multiple comparison test | | | |

According to these results, the combined use of the ADP receptor antagonist and the ACAT inhibitor demonstrated arteriosclerosis inhibitory action (synergistic effects) that was superior to the use of either alone. Thus, the pharmaceutical composition of the present invention for simultaneous administration, separate or sequential administration of the ADP receptor antagonist and the ACAT inhibitor is useful as a preventive or therapeutic agent (particularly a therapeutic agent) for arteriosclerosis or diseases caused by arteriosclerosis (particularly for arteriosclerosis).

### (Formulation 1) Tablets

2-Acetoxy-5-(α-cyclopropylcarbonyl-2-fluorobenzyl)-4,5,6,7-tetrahydrothieno[3,2-c]pyridine (10.0 mg), a sulfate of N-(1-octyl-5-carboxymethyl-4,6-dimethylindolin-7-yl)- 2,2-dimethylpropaneamide (30.0 mg), lactose (408.0 mg), cornstarch (50.0 mg) and magnesium stearate (2.0 mg) were mixed and formed into tablets with a tablet making machine to obtain tablets containing 500 mg per tablet. These tablets can be provided with a coating (preferably a sugar coating) as necessary.

### [Industrial Applicability]

The pharmaceutical composition of the present invention for simultaneous, separate or sequential administration of an ADP receptor antagonist and an ACAT inhibitor has superior inhibitory action on the occurrence of arteriosclerosis in the aorta and on the occurrence of xanthoma in the joints of the extremities, and has low toxicity. It is useful as a preventive or therapeutic agent (particularly a therapeutic agent) for arteriosclerosis or diseases derived from arteriosclerosis such as ischemic heart disease, ischemic cerebrovascular disease and peripheral vascular disease (particularly arteriosclerosis) in warm-blooded animals (particularly humans).

## Claims

1. A pharmaceutical composition for prevention or treatment of arteriosclerosis or diseases derived from arteriosclerosis comprising an ADP receptor antagonist and an ACAT inhibitor for administering simultaneously, separately or sequentially.

2. The pharmaceutical composition according to claim 1, wherein the ADP receptor antagonist is 5-[(2-chlorophenyl)methyl]-4,5,6,7-tetrahydrothieno[3,2-c] pyridine, N-[2-(methylthio)ethyl]-2-[(3,3,3-trifluoropropyl)thio]-5'-adenylic acid, monoanhydride with dichloromethylenebisphosphonic acid, 2-(propylthio)-5'-adenylic acid, monoanhydride with dichloromethylene bis(phosphonic acid), methyl (+)-(S)-α-(2-chlorophenyl)-6,7-dihydrothieno[3,2-c]pyridine-5(4H)-acetate, 2-acetoxy-5-(α-cyclopropylcarbonyl-2-fluorobenzyl)-4,5,6,7-tetrahydrothieno[3,2-c]pyridine, or a pharmaceutically acceptable salt thereof.

3. The pharmaceutical composition according to claim 1, wherein the ADP receptor antagonist is 5-[(2-chlorophenyl)methyl]-4,5,6,7-tetrahydrothieno[3,2-c]pyridine or a pharmaceutically acceptable salt thereof.

4. The pharmaceutical composition according to claim 1, wherein the ADP receptor antagonist is N-[2-methylthio)ethyl]-2-[(3,3,3-trifluoropropyl)thio]-5'-adenylic acid, monoanhydride with dichloromethylene bisphosphonic acid or a pharmaceutically acceptable salt thereof.

5. The pharmaceutical composition according to claim 1, wherein the ADP receptor antagonist is methyl (+)-(S)-α-(2-chlorophenyl)-6,7-dihydrothieno[3,2-c]pyridine-5(4H)-acetate or a pharmaceutically acceptable salt thereof.

6. The pharmaceutical composition according to claim 1, wherein the ADP receptor antagonist is methyl (+)-(S)-α-(2-chlorophenyl)-6,7-dihydrothieno[3,2-c]pyridine-5(4H)-acetate.sulfate.

7. The pharmaceutical composition according to claim 1, wherein the ADP receptor antagonist is 2-acetoxy-5-(α-cyclopropylcarbonyl-2-fluorobenzyl)-4,5,6,7-tetrahydrothieno[3,2-c]pyridine or a pharmaceutically acceptable salt thereof.

8. The pharmaceutical composition according to claim 1, wherein the ADP receptor antagonist is 2-acetoxy-5-(α-cyclopropylcarbonyl-2-fluorobenzyl)-4,5,6,7-tetrahydrothieno[3,2-c]pyridine or 2-acetoxy-5-(α-cyclopropylcarbonyl-2-fluorobenzyl)-4,5,6,7-tetrahydrothieno[3,2-c]pyridine·hydrochloride.

9. The pharmaceutical composition according to claims 1 to 8, wherein the ACAT inhibitor is 2,6-bis(1-methylethyl)phenyl N-[[2,4,6-tris(1-methylethyl)phenyl]acetyl]sulfamate, (S)-2',3',5'-trimethyl-4'-hydroxy-α-dodecylthio-α-phenylacetanilide, (-)-4-{(4R,5R)-2-[3-(2,6-diisopropylphenyl)ureidomethyl]-4,5-dimethyl-1,3-dioxolan-2-yl}phenylphosphate, N-(2,6-diisopropylphenyl)-2-tetradecylthioacetamide, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridine-3-yl]urea, N-(4,6-dimethyl-1-pentylindolin-7-yl)-2,2-dimethylpropanamide, N-(1-octyl-5-carboxymethyl-4,6-dimethylindolin-7-yl)-2,2-dimethylpropanamide, or a pharmaceutically acceptable salt thereof.

10. The pharmaceutical composition according to claims 1 to 8, wherein the ACAT inhibitor is (±)-N-(1,2-diphenylethyl)-2-(2-octyloxyphenyl)acetamide, 2,6-bis(1-methylethyl)phenyl N-[[2,4,6-tris(1-methylethyl)phenyl]acetyl]sulfamate, (1S, 2S) -2- [N- (2, 2-dimethylpropyl)-N-nonylcarbamoyl]aminocyclohexan-1-yl 3-[N-(2,2,5,5-tetramethyl-1,3-dioxane-4-carbonyl)amino]propionate, (S)-2',3',5'-trimethyl-4'-hydroxy-α-dodecylthio-a-phenylacetanilide, 2-[3-(2-cyclohexylethyl)-3-(4-dimethylaminophenyl)ureido]-4-methoxy-6-tert-butylphenol·hydrochloride, (-)-4-{(4R,5R)-2-[3-(2,6-diisopropylphenyl)ureidomethyl]-4,5-dimethyl-1,3-dioxolan-2-yl}phenylphosphate·monosodium salt, N-[2,4-bis(methylthio)-6-methyl-3-pyridyl]-2-[4-[2-(oxazolo[4,5-b]pyridin-2-ylthio)ethyl]piperazin-1-yl]acetamide, N-(2,6-diisopropylphenyl)-2-tetradecylthioacetamide, trans-1,4-bis[[1-cyclohexyl-3-(4-dimethylaminophenyl) ureido] methyl] cyclohexane, 1-benzyl-1-[3-(pyrazol-3-yl)benzyl]-3-[2,4-bis(methylthio)-6-methylpyridine-3-yl]urea, N-(4,6-dimethyl-1-pentylindolin-7-yl)-2,2-dimethylpropanamide or a sulfate of N-(1-octyl-5-carboxymethyl-4,6-dimethylindolin-7-yl)-2,2-dimethylpropanamide.

11. The pharmaceutical composition according to claims 1 to 8, wherein the ACAT inhibitor is (S)-2',3',5'-trimethyl-4'-hydroxy-α-dodecylthio-α-phenylacetanilide, (-)-4-{(4R,5R)-2-[3-(2,6-diisopropylphenyl)ureidomethyl]-4,5-dimethyl-1,3-dioxolan-2-yl}phenylphosphate, trans-1,4-bis[[1-cyclohexyl-3-(4-dimethylaminophenyl)ureido]methyl]cyclohexane, 1-benzyl-1-[3-(pyrazol-3-yl)benzyl]-3-[2,4-bis(methylthio)-6-methylpyridin-3-yl]urea, N-(1-octyl-5-carboxymethyl-4,6-dimethylindolin-7-yl)-2,2-dimethylpropanamide, or a pharmaceutically acceptable salt thereof.

12. The pharmaceutical composition according to claims 1 to 8, wherein the ACAT inhibitor is a sulfate of N-(1-octyl-5-carboxymethyl-4,6-dimethylindolin-7-yl)-2,2-dimethylpropanamide or a pharmaceutically acceptable salt thereof.

13. The pharmaceutical composition according to claims 1 to 8, wherein the ACAT inhibitor is a sulfate of N-(1-octyl-5-carboxymethyl-4,6-dimethylindolin-7-yl)-2,2-dimethylpropanamide.

14. A method for preventing or treating arteriosclerosis or a disease derived from arteriosclerosis by administering an effective amount of the pharmaceutical composition according to any of claims 1 to 13 to a warm-blooded animal.

15. The method according to claim 14, wherein the warm-blooded animal is a human.
